# EUROPEAN PATENT APPLICATION

(11) **EP 2 910 119 A1**
(43) Date of publication of application: **26.08.2015**
(21) Application number: 14156087.0
(22) Date of filing: 21.02.2014
(51) Int. Cl.: A01K 67/033, A01K 51/00

(54) **Pollen compositions and uses thereof**

(71) Applicant: Biobest Belgium NV, 2260 Westerlo (BE)
(72) Inventor: Wäckers, Felix, 2018 Antwerpen (BE); Put, Kurt, 3582 Koersel (BE); Bollens, Tim, 2230 Herselt (BE)
(74) Representative: Paemen, Liesbet R.J.

(57) **Abstract**

Provided herein are compositions for providing food supplements to predatory arthropods which are useful for the biological control of pests. Further provided herein are methods of controlling pests.

## Description

### FIELD OF THE INVENTION

Provided herein are improved compositions for providing food supplements to arthropods. Such compositions are of interest for feeding predatory arthropods such as predatory mites which are useful in the biological control of pests. Further provided herein are methods of controlling pests.

### BACKGROUND OF THE INVENTION

Plant pests such as thrips, spider mites and whitefly cause considerable damage to various crops such as salad vegetables, cut flowers and ornamental plants, resulting in significant economic loss to growers and higher prices for consumers. Although plant pests may be controlled using chemical pesticides, this is not always possible or desirable. Indeed, the widespread use of chemical pesticides can result in health and environmental problems, and in the appearance of resistant insect varieties.

Alternative methods for plant pest control have been developed, such as the use of natural predators of plant pests. In particular, beneficial arthropods such as predatory mites are the cornerstone of the biological control strategy. Predatory mites such as *Amblyseius swirskii, A. cucumeris* and *A. californicus* are widely used to control pests like thrips, whitefly and spider mite. The predatory mites may be applied to the plants manually, via controlled release systems, or via other methods. Predatory mites also have applications in areas of pest control other than crop protection, such as in the protection of animals, animal products or fabrics/carpets.

Nevertheless, there are situations - often linked to a lack of food supply - in which these beneficials struggle or even fail to establish in the crop. This is a serious bottleneck to the success of biological pest control in a range of crops and plant nurseries. It is known that predatory mites can be reared using alternative diets such as pollen. Accordingly, pollen may be applied to plants in order to sustain predatory mite populations which are also present on the plants. Such application may be done by spraying pollen onto the crops. However, pollen is relatively expensive to use because of the labour intensive collection process. Moreover, the tendency of pollen to clump together and its low shelf-life can significantly impede the spraying of pollen.

Accordingly, there is a need for improved pollen compositions.

### SUMMARY OF THE INVENTION

Provided herein are compositions for providing food supplements to arthropods such as predatory mites which may be useful in the biological control of pests such as plant pests. Indeed it has been observed that the compositions provided herein allow improved handling of pollen for use as a food supplement. Further provided herein are methods of controlling pests using said compositions.

Thus provided herein are improved compositions comprising pollen. In particular embodiments, the compositions envisaged herein comprise between 1 w% and 99 w% pollen. In further particular embodiments, the compositions comprise between 25 w% and 99 w% pollen, most preferably between 40 w% and 95 w% pollen and one or more water absorbing materials.

In particular embodiments, the compositions envisaged herein comprise between 25 w% and 95 w% pollen and between 75 w% and 5 w% of the one or more water absorbing materials. In certain embodiments, the one or more water absorbing materials are selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, seed husks, superabsorbent polymer, hydroxypropyl methylcellulose, attapulgite, montmorillonite, talc, diatomaceous earth, silica gel, fumed silica, molecular sieves (such as zeolites), calcium sulfate, and calcium chloride. In further embodiments, the water absorbing material comprises brine shrimp cysts.

In particular embodiments, the one or more water absorbing materials are provided as particles having a minimal size of at least 20 µm.

Further provided in this application is the use of the compositions comprising between 1 w% and 99 w% pollen and one or more hygroscopic excipients, as a nutritional source for arthropods, more particularly predatory arthropods. In particular embodiments, the predatory arthropods are predatory mites. In particular embodiments the food compositions are of interest as a nutritional source for predatory mites of a genus selected from the group consisting of *Euseius, Amblyseius, Neoseiulus, Iphiseius, Indoseiulus, Kampimodromus, Typhlodromalus, Phytoseius* and *Typhlodromus.*

Accordingly, the present application provides methods of sustaining a population of arthropods on a substrate of interest by applying to said substrate of interest the compositions envisaged herein. More particularly the methods comprise applying to said substrate a composition comprising between 1 w% and 99 w% pollen and one or more excipients, comprising one or more water absorbing materials as described herein. The methods further encompass the step of allowing the arthropods to feed on the pollen in said composition.

In particular embodiments of the methods of sustaining arthropods such as predatory mites envisaged herein, the one or more excipients are provided as particles having a minimal size of 20 µm and a maximal size of 500 µm. In certain embodiments, the compositions comprise less than 10 w% water.

In certain embodiments, the substrate on which the compositions envisaged herein are applied as a food source for arthropods is a plant of interest. In further embodiments of the methods envisaged herein which involve applying pollen compositions to a plant of interest, the pollen provided in the compositions envisaged herein is obtained from a plant species other than said plant of interest.

In particular embodiments, in the methods and uses envisaged herein the composition is applied onto a substrate of interest at least once per month, for at least three months.

The present application further provides methods of controlling pests on a substrate of interest, which methods comprise the steps of providing said substrate with a population of predatory arthropods for said pests, applying to said substrate a composition comprising between 1 w% and 99 w% pollen, and one or more water absorbing materials and allowing said predatory arthropods to feed on said pollen.

The methods described herein can assist in maintaining populations of predatory arthropods on plants or other objects to be protected from pests, even in the absence of prey. In particular embodiments, the compositions described herein may be easily applied to plants or other objects via dusting or spraying. The above and other characteristics, features and advantages of the concepts described herein will become apparent from the following detailed description, which illustrates, by way of example, the principles of the invention.

### DETAILED DESCRIPTION OF THE INVENTION

While potentially serving as a guide for understanding, any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to ensure one or more of the technical effects envisaged herein. It is to be understood that each value as used herein is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are also envisaged herein, and form different embodiments, as would be understood by those in the art. For example, in the appended claims, any of the features of the claimed embodiments can be used in any combination.

### Composition comprising pollen

Provided herein are compositions for providing pollen as a nutritional source for arthropods. More particularly the pollen compositions are envisaged as food supplements for predatory mites which in the context of the biological control of (plant) pests. More particularly, the compositions according as envisaged herein comprise pollen and one or more excipients, which compositions allow facilitated handling. This will be explained more in detail herein below.

The compositions described herein comprise pollen as a main ingredient. The pollen may be from one or more plant species. The pollen contains nutrients for arthropods. More particularly the pollen can be used as a food source for arthropod predators and parasitoids, which often require or can be maintained on plant-provided food at least during part of their life cycle. For instance, many predatory mite species are known to be able to establish and maintain on a supply of pollen in the absence of prey species.

The amount of pollen in the composition is not critical to the invention. However, typically, the amount of pollen and excipient is balanced to ensure sufficient nutrient value and excipient function in the composition.

In certain embodiments, the compositions as envisaged herein comprise at least 1 w%, at least 5 w%, at least 10 w%, at least 20 w%, at least 30 w%, at least 40 w%, at least 50 w%, at least 60 w%, or at least 70 w% of pollen grains. For obtaining optimal nutrient values for the uses envisaged herein, it is preferred that the compositions comprise at least 15 w% of pollen grains, even more preferably at least 20 w%.

In certain embodiments, the composition comprises at most 99 w%, at most 95 w%, at most 90 w%, at most 85 w%, at most 80 w%, or at most 75 w% of pollen grains.

In particular embodiments, the composition comprises between 1 w% (percent by weight) and 99 w% of pollen grains. In further embodiments, the composition comprises between 25 w% and 99 w% pollen, more particularly between 25 w% and 95 w%. In yet further embodiments, the composition comprises between 30 w% and 90 w% pollen, more particularly between 30 w% and 80 w%. In specific embodiments, the composition comprises between 75 w% and 99 w% pollen. Such compositions enable the application of a suitable amount of pollen to a substrate to maintain an arthropod population, such as a predatory mite population.

Many plants are known to provide pollen on which arthropods can feed. For use in the context of predatory mites, in particular embodiments, the pollen provided in the compositions envisaged herein are pollen from cattail (genus *Typha),* as these pollen are relatively insensitive to humidity. Other suitable pollen include, but are not limited to cherry, apricot, walnut, hazel, birch, apple, pear, plum, almond, maize, oak, *Hirschfeldia incana, Mesembryanthenum, Ricinus communis, Malephora crocea,* and *Brevipalpus chilensis* pollen.

In particular embodiments, the pollen may be fresh. However, it is envisaged that in certain embodiments, the pollen may be treated. For example, the pollen may be dried (see further) and/or frozen. This can significantly increase the shelf-life. In certain embodiments, the pollen may be sterilized, for example via a treatment with UV irradiation.

Under natural conditions, pollen usually takes up moisture from the environment. Fresh pollen may contain over 50 w% water. However, under moist conditions, pollen may rapidly decline in food quality and clump together, thus reducing the suitability of the pollen for feeding predatory mites. Thus, in certain embodiments, the pollen may be (partially) dehydrated. Preferably, the pollen used in the preparation of the composition comprises less than 20 w% water, preferably less than 15 w% water, more preferably less than 10 w% water.

It is will further be understood to the skilled person that, as detailed above the addition of water is detrimental maintaining the quality of the pollen. Accordingly it is further preferred that the compositions as provided herein do not comprise added water.

In preferred embodiments, the compositions described herein can be sprayed or dusted. To this end, in particular embodiments, the composition does not contain large clumps of pollen grains. Although the inventors have found that the mixing of pollen with absorbents as envisaged herein may in some cases partially reverse clumping (see examples), it is preferred that the pollen are not clumped prior to the preparation of the pollen composition described herein.

Accordingly, honeybee collected pollen, which typically forms large clumps, are therefore not preferred for these embodiments. In certain embodiments, the pollen is provided as a powder containing individual pollen grains, or small aggregates of pollen grains. The term "small aggregates" as used herein refers to aggregates containing less than 50 pollen grains, preferably less than 30 pollen grains, more preferably less than 20 pollen grains, and most preferably less than 10 pollen grains. More particularly, the pollen are provided as particles (individual grains and/or small aggregates thereof) having a maximal particle size of 200 µm. In further embodiments, the maximal particle size is 100µm, preferably 50µm, and more preferably 20µm.

The particle size of pollen grains and/or excipient particles can be determined via a dry sieve test, according to the ISO 3310 and ISO 565 standards. The particle size of a collection of particles can be expressed as "*Dₓ*", which is the theoretical sieve opening of a sieve, having such a mesh size that x wt% of the particles passes through the sieve. As used herein, the term "average particle size" refers to D*₅₀*; the term "maximal particle size" refers to *D₁₀₀*; and the term "minimal particle size" refers to Do. If the present application refers to particles having a particle size between two values, these values are Do and *D₁₀₀.*

In addition to pollen, the compositions described herein comprise one or more excipients which include a composition which functions as an adsorbent. The inventors have found that the addition of an absorbent to pollen can significantly reduce aggregation of the pollen grains and may help conserving the nutritional value of the pollen. Aggregation can be particularly problematic when pollen is frozen. Indeed, the pollen can become moist upon defrosting, causing aggregation of the pollen grains and a rapid decline in food quality. Thus, it has been found that the use of an adsorbent can significantly improve the quality and/or shelf-life of pollen, without interfering with its function, i.e. as a nutritional source for arthropods.

Thus, in preferred embodiments, the excipient comprises or consists of one or more absorbents, more particularly water absorbing materials. As used herein, the term "water absorbing material" may encompass, without limitation, hygroscopic materials. A hygroscopic material is one having the property of readily imbibing moisture from the atmosphere. In particular embodiments, the water absorbing material(s) used in the envisaged compositions are able to take up water at a temperature of about 25°C in an amount of at least 10 grams per 100 grams of material, and preferably in an amount of at least 20 grams per 100 grams of material.

In particular embodiments, the composition comprises at least 5 w% absorbents. In further embodiments, the composition comprises at least 10 w%, at least 20 w%, at least 30 w%, at least 40 w%, at least 50 w%, or more absorbents, and optionally one or more other excipients.

In certain embodiments, the one or more excipients comprise at least 50 w% absorbents.

In certain embodiments, the one or more absorbents are selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, seed husks, superabsorbent polymer, hydroxypropyl methylcellulose, attapulgite, montmorillonite, talc, diatomaceous earth, silica gel, fumed silica, molecular sieves (such as zeolites), calcium sulfate, and calcium chloride.

In certain embodiments, the one or more absorbents are selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, seed husks, and talc.

In certain embodiments, the one or more absorbents are biodegradable materials selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, and seed husks.

In further embodiments, the one or more absorbents may be selected from brine shrimp cysts, talc or flour. Suitable flour types include, but are not limited to rice flour, barley flour, rye flour, barley-bran flour, barley-flour, wheat flour. Rice flour is particularly preferred. Suitable plant meal includes, but is not limited to soybean meal, corn meal, cotton seed meal, and linseed meal. Suitable vegetable fibers include, but are not limited to cotton fibers, hemp fibers, sisal fibers, flax fibers (linen), jute fibers, corn fiber, oat fiber, pea fiber, rice fiber, soy fiber, and sugar beet fiber. Suitable seed hulls include, but are not limited to rice hulls, rice bran, wheat bran, corn bran, oat bran, cotton seed hulls, and grain bran. Preferred seed hulls include rice bran, and wheat bran.

The term "superabsorbent polymer" as used herein refers to a polymer which is substantially water-insoluble and preferably water-gelling, which absorbs and is capable of holding at least about ten times its own weight of water or aqueous fluid. Such polymers are typically (lightly) crosslinked polymers, preferably lightly crosslinked hydrophilic polymers. Examples of superabsorbent polymers include crosslinked sodium polyacrylate and carboxymethylcellulose.

The above-described materials typically have suitable water absorbing properties. In addition, they are safe to apply to crops, which is of interest for particular uses of the compositions envisaged herein.

In particular embodiments of the composition described herein, the absorbent may comprise brine shrimp cysts. Brine shrimps are aquatic crustaceans of the genus *Artemia.* Accordingly, brine shrimp cysts are also referred to herein as *"Artemia* cysts". *Artemia* cysts are eggs which can be dried and stored and then hatched years later by returning them to salt water. *Artemia* cysts typically have a (bi)concave to spherical shape, depending on the degree of hydration. Indeed, dehydrated cysts typically have a biconcave or concave shape. Preferably, the cysts are provided in a dehydrated state. The cysts typically have a size between 200 µm to 300 µm. *Artemia* cysts are suitable for use as an absorbent, as they are hygroscopic and able to take up relatively high volumes of water. The inventors have moreover found that the cysts and/or embryos contained therein may provide nutrients to arthropods such as predatory mites. Accordingly, the presence of *Artemia* cysts may not only protect the pollen from moisture, but may also increase the nutritional value of the composition. The *Artemia* cysts may further facilitate the adhesion of the pollen to a substrate. In particular, the *Artemia* cysts may facilitate the adhesion of the pollen to plant parts such as leafs.

In particular embodiments, the composition comprises at least 5 w%, at least 10 w%, or at least 15 w% *Artemia* cysts.

In particular embodiments, the water absorbing materials as described herein may also serve as a carrier for the pollen.

In particular embodiments, the one or more excipients of the composition described herein may comprise one or more fillers or carriers in addition to the one or more water absorbing materials. This may facilitate the controllable application of pollen grains onto a surface. Examples of suitable fillers are light particulate or granular materials such as (activated) carbon, vermiculite, powdered casein, powdered walnut shells, buckwheat shells, and powdered egg albumin.

In particular embodiments, the composition comprises between 1 w% and 80 w% fillers. In certain embodiments, the composition comprises at least 10w% fillers. In further embodiments, the composition comprises between 10 w% and 75 w% fillers. In certain embodiments, the composition comprises at least 20w%, at least 30 w%, at least 40 w%, at least 50 w%, or more fillers.

In particular embodiments the composition described herein comprises in addition to pollen, other nutritional sources for arthropods. Accordingly, in specific embodiments, the one or more excipients may comprise, in addition to the one or more water absorbing materials one or more (non-pollen) nutritional sources.

In specific embodiments, the nutritional source(s) is or comprises a sugar source. Preferably, the sugar source may comprise one or more sugars or sugar alcohols selected from sucrose, fructose, glucose, maltose, trehalose, galactose, raffinose, mannitol, and sorbitol. The sugar source is preferably provided in a solid form, e.g. as a powder and/or crystalline particles. In particular embodiments, the composition described herein comprises between 0.5 w% and 5 w% of (added) sugars. However, it is envisaged that in certain embodiments, the composition does not comprise added sugars.

Where the compositions are envisaged for use in predatory arthropods, the additional or non-pollen nutritional source may include prey and/or non-prey nutritional sources. Suitable nutritional sources include, but are not limited to prey insects such as spider mites or other prey mites, (dried) insect eggs, and/or artificial diets. The preparation of dried insect eggs may be performed as described in international patent application WO 2011/010308.

Additionally or alternatively, in certain embodiments the excipients of the compositions envisaged herein may comprise one or more fibrous materials. This fibrous material may provide an oviposition substrate for the female arthropods. Where the compositions envisaged herein are for use as a nutritional source for predatory mites, the addition of suitable oviposition substrates for rearing mites can be envisaged. Such materials have been described in the art and include for example (chopped) cotton fibers.

The one or more excipients of the compositions envisaged herein typically contain only minimal amounts of water. More particularly, the composition (i.e. pollen and excipients together) preferably contains less than 20 w% water, or even less than 10 w% water. In certain embodiments, the composition contains less than 5 w%, less than 3 w%, or less than 1 w% water. This may suppress the decline in food quality of the pollen, and may prevent clumping of the pollen grains.

In particular embodiments, the compositions described herein are envisaged for use in the biological control of pests, including but not limited to plant pests (see further). Accordingly, for these embodiments, it is preferred that the application of the composition on a substrate such as a plant does not cause health and/or environmental problems. Therefore, the one or more excipients envisaged for use in these embodiments are preferably selected from biodegradable materials and inert minerals.

It will be understood from the above that the excipients as described herein do not comprise pollen, but can be mixed with the pollen grains. Accordingly, the composition may be provided as a mixture of pollen grains and one or more excipients other than pollen.

In certain embodiments, the composition comprises at least 1 w%, at least 5 w%, at least 10 w%, at least 20 w%, at least 30 w%, at least 40 w%, or at least 50 w% of excipient(s) such as those described hereinabove.

In certain embodiments, the composition comprises at most 99 w%, at most 95 w%, at most 90 w%, at most 85 w%, at most 80 w%, or at most 75 w% of excipient(s).

In particular embodiments, the composition comprises between 25 w% and 95 w% pollen, and between 5 w% and 75 w% excipients. In further embodiments, the composition comprises between 25 w% and 90 w% pollen, and between 10 w% and 75 w% excipients. In further embodiments, the composition comprises between 30 w% and 80 w% pollen, and between 20 w% and 70 w% excipients.

While as described above, different types of excipients are envisaged, excipients generally are solid materials and are preferably provided in a particulate form, e.g. as granules and/or a powder. This facilitates application of the composition on a substrate. In preferred embodiments, the excipients are provided as particles having a maximal particle size below 500 µm, preferably below 250 µm, or below 200 µm. Such particle sizes facilitate the spraying or dusting of the composition.

In particular embodiments however, the excipients are preferably provided as particles having a minimal size of at least 10 µm, at least 20 µm, at least 30 µm, at least 50 µm, at least 75 µm, or at least 100 µm. Indeed, although the particles are in principle as small as possible for spraying or dusting the composition, the inventors have found that the use of particles having a size similar to or smaller than the size of individual pollen grains as excipient in the composition can have an adverse effect on the suitability of the composition for feeding the arthropods. Accordingly, in these embodiments, a larger particle size (i.e. t least 10µm) can be envisaged. In further embodiments, the excipients are provided as particles having a size between 20 µm and 500 µm. In further embodiments, the excipients are provided as particles having a size between 50 µm and 400 µm.

The application further envisages that the compositions provided herein may be provided in combination with additional components, for use in the context of specific applications. For instance it can be envisaged that compositions are provided which contain both the arthropods of interest and the pollen compositions as envisaged herein as their nutritional source. Thus, for instance in particular embodiments, the application provides compositions comprising, in addition to the pollen and one or more excipients as detailed above, a population of predatory mites. Such compositions are of interest for use in pest control. More particularly such a composition can be applied in the protection of objects which are not yet provided with predatory mites, or with only a low amount of predatory mites. However, it is envisaged that in certain embodiments, the composition does not comprise the arthropod for which the nutritional source is intended. Compositions without live arthropods can typically be stored longer, e.g. by freezing the composition and/or storing the composition in a sealed container.

In particular embodiments, the composition described herein consists of a mixture of pollen and one or more absorbents. The composition can be used as such for feeding predatory mites, or may be mixed with further excipients (such as fillers and/or nutritional sources).

The application further envisages containers and dispensing devices comprising the compositions disclosed herein.

Dispensing devices for dispensing pollen onto objects such as plants or fabrics are known to the skilled person. In particular embodiments, the dispensing device may comprise a pollen storage container configured to contain pollen. Ready-for-use containers or dispensing devices comprising the compositions as envisaged herein are also provided.

### Use of the compositions

Further provided herein is the use of the compositions described herein, as a nutritional source for arthropods. More particularly, the application envisages methods for feeding predatory arthropods, and/or methods for protecting objects against pests by providing the compositions envisaged herein as a nutritional source for predatory arthropods.

The compositions described herein may thus be used as a nutritional source in the rearing of arthropods, such as predatory arthropods. The term "rearing" as used herein broadly refers to breeding, reproducing, surviving and growing of arthropods, and includes the propagation and increase of population by means of sexual reproduction. Such use typically involves providing the composition to a rearing population, i.e. a population which is capable of increasing the number of its individuals by means of sexual reproduction. A rearing population may comprise sexually mature adults from both sexes, and/or individuals of both sexes of immature life stages (such as eggs and/or nymphs) which can mature to sexually mature adults. Additionally or alternatively, the rearing population may comprise one or more fertilized females.

Accordingly provided herein are methods of sustaining a population of arthropods on an object, using a composition as described herein above. The methods described herein involve applying the composition to an object, and allowing the arthropods to feed on the pollen comprised in the applied composition. In particular embodiments the arthropods are predatory mites. This will be explained further herein below.

The methods described herein may be used for protecting an object from a pest by sustaining on said object a population of an arthropod which is a predatory arthropod for said pest. More particularly, the predatory arthropod population is a predatory mite population. In particular embodiments, the object is an object for which pest control via predatory mites is desired. Suitable objects include but are not limited to plants, animals, animal products, and fabrics or fibre-based materials such as carpets. The use of predatory arthropods is particularly suitable for protecting plants or crops. Exemplary crops for which such methods are considered suitable include vegetable crops such as peppers, eggplants, cucumbers, melons, watermelons, grapes, strawberries, raspberries, ornamental crops (e.g. roses), or tree crops (e.g. apple trees, *Citrus* spp.).

The application of the compositions described herein is particularly useful when controlling pests which are preys of predatory mites, such as spider mites, thrips, and whitefly. In particular embodiments, the pest comprises thrips and/or (spider) mites. In certain embodiments, the pest comprises thrips. Exemplary thrips species include Western Flower Thrips *(Frankliniella occidentalis)* and Onion thrips *(Thrips tabaci).* Exemplary mites include two-spotted spider mites *(Tetrancychus urticae),* red mites *(Dermanyssus gallinae),* cured ham mites *(Tyrophagus putrescentiae)* and dust mites *(Dermatophagoides farinae* and *Dermatophagoides pteronyssinus).* Thus the compositions provided may be used in methods for controlling each or all of these mites.

In these applications, the compositions described herein can improve methods for the (biological) control of pests, such as those using predatory mites. Accordingly, particular embodiments of the methods envisaged herein are methods for sustaining a population of predatory mites on an object and/or methods for protecting an object from a pest using the compositions described herein as a nutritional source for predatory mites. In these methods, the object may already be provided with a population of predatory mites. Additionally or alternatively, the method may comprise the step of introducing predatory mites on or near the object. In particular embodiments, methods for controlling pests are provided which involve the step of introducing predatory mites on or near the object. The term "introducing" as used herein in the context of introducing predatory mites, refers to the introduction of predatory mites on or near a crop to be protected from pests.

In preferred embodiments, the predatory mites are phytoseiid predatory mites, i.e. members of the family *Phytoseiidae.* In certain embodiments, the predatory mites are from a genus selected from the group consisting of *Euseius, Amblyseius, Neoseiulus, Iphiseius, Indoseiulus, Kampimodromus, Typhlodromalus, Phytoseius* and *Typhlodromus.* These mites are typically generalist mites which can be reared using pollen as food source. In particular embodiments, the predatory mites are from the genus *Euseius.* Nonlimiting examples of mite species suitable for use in the presently disclosed rearing methods include *Euseius stipulatus, Amblyseius cucumeris, Typhlodromus pyri, Amblyseius swirskii,* and *Iphiseius degenerans.*

The methods for sustaining a predatory mite population and/or for protecting an object described herein comprise the step of applying a composition as described herein to the object. The pollen composition can be applied to the object before, after, or simultaneous with the introduction of the predatory mites. In particular embodiments, the predatory mites are introduced to the object prior to the application of the pollen composition.

The method used for applying the compositions envisaged herein to an object is not critical. In the methods envisaged herein, the composition is provided to the object in such a way that the arthropods present or provided on or near the object are allowed to feed on the composition. The pollen composition described herein can be applied onto objects using various methods known in the art, such as spraying, dusting or brushing. In preferred embodiments, the composition is applied to the object by dusting or spraying.

In the methods for sustaining a predatory mite population and/or for protecting objects as described herein, the composition may be applied once or repeatedly. In particular embodiments, the composition is provided repeatedly or regularly. In particular embodiments, the composition is applied to the object at least once per month, for at least three months. In certain embodiments, the composition is applied at a frequency ranging from once per day to once per fortnight. In particular embodiments, the composition is provided once per week.

The amount of the composition to be provided on an object in the different methods envisaged herein may depend on the pollen type and amount of pollen contained in the composition, and on the predatory mite species. In general, an amount ranging between 0.01 g and 1 g of pollen per 1000 mites per week is sufficient. In particular embodiments, between 0.01 g and 0.5 g of pollen are added per 1000 mites and per week, for example about 0.05 g or about 0.1 g of pollen per 1000 mites and per week.

In particular embodiments, the object envisaged to be protected and/or for use in maintaining the predatory mite composition is a plant bearing blossoms. In such embodiments, it is preferred that the application of the composition on the plant does not interfere with pollination of the plant. Therefore, in certain embodiments, the pollen is obtained from one or more plant types other than that of the plant to be protected. Interference with pollination may further be prevented by applying the composition on non-reproductive parts of the plant such as leaves or stems, and/or applying the composition outside the flowering period of the plant to be protected. Accordingly, in particular embodiments, the plant to be protected does not bear blossoms upon application of the composition.

Through the application of the pollen composition on an object, the pollen comprised in the composition is made accessible to the arthropod present on or near the object. The methods described herein thus involve allowing the arthropod to feed on the pollen. This helps the arthropod population to survive, especially when the amount of an alternative nutritional source, such as prey present on the object is low.

### EXAMPLES

The following examples are provided for the purpose of illustrating the present invention and by no means are meant and in no way should be interpreted to limit the scope of the present invention.

A number of pollen compositions were prepared by mixing pollen with an excipient selected from talc powder, brine shrimp cysts, and rice flour; using various pollen/excipient weight ratios. Table 1 provides an overview of the compositions which were prepared.

**Table 1 - experimental results obtained using various pollen compositions**

| **Experiment** | **Excipient** | **w% pollen** | **w% absorbant** | **Time (h)** | **Spreading distance (m)** | **Clumping** |
|---|---|---|---|---|---|---|
| 1 | n/a | 100 | 0 | 1 | 6 | very high |
| 2 | n/a | 100 | 0 | 24 | 7 | high |
| 3 | talc | 95 | 5 | 24 | 7 | low |
| 4 | talc | 67 | 33 | 1 | 7 | none |
| 5 | talc | 67 | 33 | 24 | 7 | low |
| 6 | talc | 33 | 67 | 1 | 8 | none |
| 7 | talc | 33 | 67 | 24 | 8 | none |
| 8 | talc | 20 | 80 | 24 | 7 | none |
| 9 | cysts | 95 | 5 | 1 | 7.5 | medium |
| 10 | cysts | 95 | 5 | 24 | - | low-medium |
| 11 | cysts | 90 | 10 | 1 | 6.5 | low-medium |
| 12 | cysts | 90 | 10 | 24 | - | low |
| 13 | cysts | 50 | 50 | 1 | 7.5 | low |
| 14 | cysts | 50 | 50 | 24 | 7.5 | none |
| 15 | rice flour | 95 | 5 | 1 | 8 | low-medium |
| 16 | rice flour | 95 | 5 | 24 | - | low |
| 17 | rice flour | 90 | 10 | 1 | 8 | low-medium |
| 18 | rice flour | 90 | 10 | 24 | - | low |
| 19 | rice flour | 80 | 20 | 1 | 8.5 | low |
| 20 | rice flour | 80 | 20 | 24 | - | low |

For all compositions, cattail pollen was used. 20g of each composition was prepared by combining appropriate amounts of pollen and excipient in a recipient, followed by shaking until a homogeneous mixture was obtained. Within one hour of preparation, a part of each composition was sprayed using a spray gun. The remainder of the composition was sprayed about 24 hours after preparation. The results of the spray test are also provided in Table 1. For the experiments using rice flour, the spray tests were only performed 1 hour after preparation.

In general, all of the compositions could be sprayed. However, for some compositions spraying was difficult due to clumping of the pollen grains. More particularly, it was found that pure fresh pollen without excipient have a high tendency to aggregate, resulting in clumping. The recipient containing the composition needed to be tapped heavily to obtain an acceptable pollen release. The spraying distance therefore only partially reflects the spraying difficulty. It is further noted that the spraying properties of (non-clumped) pollen compositions may be further improved through the addition of suitable fillers or carriers as described herein.

Significantly less clumping was observed when the compositions comprise talc powder as an excipient. One hour after preparation, no clumping was observed with the compositions comprising 20, 33, and 67 w% pollen. These compositions could be readily released from the recipient without tapping. Some clumping was observed at high pollen content (95w%). A slight increase of the clumping was observed after 24 hours for the composition comprising 67 w% pollen.

The use of brine shrimp cysts and rice flour yields similar results as the use of talc powder directly after preparation. No clumping was observed in the mixture comprising 50w% brine shrimp eggs, even when using clumped pollen for the preparation of the mixture. Clumping was still observed in the compositions comprising 80w% or more pollen. However, the degree of clumping was reduced after 24 hours. Clumping of the 90w% pollen/10w% brine shrimp cysts could be reduced further by mixing the composition with an equal volume of saw dust.

From the above results, it is clear that the addition of excipients such as talc powder, brine shrimp cysts, rice flour, and saw dust can reduce clumping and can significantly facilitate spraying or other handling of pollen. In general, the tendency of the pollen to clump reduces with the amount of excipient added. Even a small amount of a few w% can reduce pollen clumping, whereas larger amounts such as 50 w% may allow for obtaining clump-free compositions.

Surprisingly, the use of brine shrimp cysts and rice flour results in compositions which show a reduction of the number of clumps upon ageing.

## Claims

1. A method of sustaining a population of predatory mites on a substrate of interest comprising:
- applying to said substrate of interest a composition comprising
• between 1 w% and 99 w% pollen; and
• one or more excipients, comprising one or more water absorbing materials and
- allowing said predatory mites to feed on said composition comprising said pollen.

2. The method according to claim 1, wherein said one or more water absorbing materials are selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, seed husks, superabsorbent polymer, hydroxypropyl methylcellulose, attapulgite, montmorillonite, talc, diatomaceous earth, silica gel, fumed silica, molecular sieves (such as zeolites), calcium sulfate, and calcium chloride.

3. The method according to claim 1 or 2, wherein said water absorbing material comprises brine shrimp cysts.

4. The method according to any one of claims 1 to 3, wherein said substrate of interest is a plant.

5. The method according to claim 4, wherein said pollen is obtained from a plant species other than said plant of interest.

6. The method according to any one of claims 1 to 5, wherein said one or more excipients are provided as particles having a minimal size of 20 µm and a maximal size of 500 µm.

7. The method according to any one of claims 1 to 6, wherein said pollen comprises less than 10 w% water.

8. The method according to any one of claims 1 to 7, wherein said composition is applied onto said substrate of interest at least once per month, for at least three months.

9. A method of controlling pests on a substrate, comprising:
- providing said substrate with a population of predatory mites;
- applying to said substrate a composition comprising between 1 w% and 99 w% pollen, and one or more water absorbing materials; and
- allowing said predatory mites to feed on said composition comprising said pollen.

10. The method according to any one of claims 1 to 9, wherein said composition comprises
- between 25 w% and 95 w% pollen; and
- between 75 w% and 5 w% of said one or more water absorbing materials.

11. A composition comprising between 25 w% and 99 w% pollen, and one or more water absorbing materials.

12. The composition according to claim 11, wherein said one or more water absorbing materials are selected from the list consisting of brine shrimp cysts, sawdust, vegetable fibers, seed hulls, starch, (powdered) cellulose, cellulose fibers, fluff pulp, paper, flour, plant meal, peat moss, corn cob, fish meal, grain germ, seed husks, superabsorbent polymer, hydroxypropyl methylcellulose, attapulgite, montmorillonite, talc, diatomaceous earth, silica gel, fumed silica, molecular sieves (such as zeolites), calcium sulfate, and calcium chloride.

13. The composition according to claim 11 or 12, wherein said one or more water absorbing materials are provided as particles having a minimal size of at least 20 µm.

14. Use of a composition comprising between 1 w% and 99 w% pollen and one or more hygroscopic excipients as a nutritional source for predatory mites.

15. The use according to claim 14, wherein said predatory mites are from a genus selected from the group consisting of *Euseius, Amblyseius, Neoseiulus, Iphiseius, Indoseiulus, Kampimodromus, Typhlodromalus, Phytoseius* and *Typhlodromus.*
